Europäisches Patentamt

European Patent Office

Office européen des brevets

(19)

(11) Veröffentlichungsnummer: **0 170 979**
**A2**

(12) # EUROPÄISCHE PATENTANMELDUNG

(21) Anmeldenummer: **85109263.5**

(22) Anmeldetag: **24.07.85**

(51) Int. Cl.⁴: **A 61 K 47/00,** A 61 K 31/71, A 61 L 15/03, A 61 L 27/00

(30) Priorität: **07.08.84 DE 3429038**

(43) Veröffentlichungstag der Anmeldung: **12.02.86 Patentblatt 86/7**

(84) Benannte Vertragsstaaten: **AT BE CH DE FR GB IT LI NL SE**

(71) Anmelder: **Merck Patent Gesellschaft mit beschränkter Haftung, Frankfurter Strasse 250, D-6100 Darmstadt (DE)**
Anmelder: **INTERMEDICAT GMBH, Gerliswilstrasse 74, CH-6020 Emmenbrücke (CH)**

(72) Erfinder: **Fleckenstein, Peter, Dr., Am Bürgerhaus 4, D-3501 Fuldabrück 1 (DE)**
Erfinder: **Wahlig, Helmut, Dr., Roemheldweg 16, D-6100 Darmstadt (DE)**
Erfinder: **Dingeldein, Elvira, Dr., Am Spitzenpfad 9, D-6072 Dreieich (DE)**

(54) **Wirkstoffdepot.**

(57) Die Anmeldung betrifft ein resorbierbares Wirkstoff-Depot auf Basis von Kollagen, dadurch gekennzeichnet, daß ein rekonstituiertes Kollagen mit Vlies- oder Schwammstruktur mit einem Aminoglykosid-Antibiotikum und einem bioresorbierbaren Polymeren mit polyanionischem Charakter beladen ist.

ACTORUM AG

## Wirkstoff-Depot

Die Erfindung betrifft ein resorbierbares Wirkstoff-Depot auf Basis von Kollagen.

Es sind bereits zahlreiche Materialien als Träger für medizinische Wirkstoffe vorgeschlagen worden, die in den Körper eingebracht werden können und dort während oder nach der Freisetzung des Wirkstoffs resorbiert werden, so daß eine nachträgliche Entfernung des Trägers entfallen kann. In der Regel sind es synthetische organische Polymere, es sind aber auch Implantate auf Basis von Kollagen vorgeschlagen worden.

So sind in der DOS 2843963 im Körper resorbierbare geformte Massen beschrieben, wobei pulverförmiges denaturiertes Kollagen zusammen mit einem Wirkstoff und einem Bindemittel zu Formkörpern verpreßt ist. In der EP-OS 69 260 wird eine wirkstoffhaltige blattförmige Kollagen-Einlage beschrieben, die ggf. zu einer Stabform aufgewickelt wird. Zur Herstellung dieses Kollagens wird eine Kollagen-Lösung verwendet, die gefriergetrocknet wird.

Alle diese Zubereitungen sind jedoch noch nicht optimal in bezug auf die Freisetzung des Wirkstoffs, die Verträglichkeit und das Resorptionsverhalten des Trägers. Es bestand deshalb die Aufgabe, ein Wirkstoff-Depot zu finden, mit dem insbesondere Aminoglykosid-Antibiotika so in den Körper eingebracht werden können, daß der Wirkstoff über einen ausreichend langen Zeitraum in ausreichend hoher Konzentration freigesetzt wird, wobei das Depot gut verträglich ist und gut resorbiert wird.

Es wurde nun gefunden, daß ein aus rekonstituierten Kollagen-Fasern aufgebautes Wirkstoff-Depot von vliesartiger oder schwammiger Struktur, dessen kollagener Träger zum einen körperverträglich und vollständig resorbierbar ist und zum anderen eingelagerte Wirkstoffe von Aminoglykosid-Charakter protrahiert freizusetzen vermag, dadurch gewonnen werden kann, daß man in die Kollagen-Matrix zusätzlich ein bioresorbierbares Polymeres mit polyanionischem Charakter einbringt.

Gegenstand der Erfindung ist daher ein resorbierbares Wirkstoff-Depot auf Basis von Kollagen, das dadurch gekennzeichnet ist, daß ein rekonstituiertes Kollagen mit Vlies- oder Schwammstruktur mit einem Aminoglykosid-Antibiotikum und einem bioresorbierbaren Polymeren mit polyanionischem Charakter beladen ist.

Gegenstand der Erfindung ist auch ein Verfahren zur Herstellung eines resorbierbaren Antibiotikum-Depots, das dadurch gekennzeichnet ist, daß zu einer Suspension eines rekonstituierten Kollagens, in dem die native Molekülstruktur weitgehend erhalten ist, mit einer Faserlänge von max. 10 mm, ein Aminoglykosid-Antibiotikum und ein bioresorbierbares Polymeres mit polyanionischem Charakter gegeben wird, vor oder nach dieser Zugabe der pH-Wert der Suspension auf einen Wert unterhalb 7 eingestellt wird und die Suspension, ggf. nach Eingießen in Formen, gefriergetrocknet wird.

Zur Gewinnung des Ausgangsmaterials für die erfindungsgemäßen Depots können aus der Literatur bekannte Verfahren herangezogen werden, bei denen das aus Biomaterialien,
wie tierischen Häuten oder Sehnen gewonnene Kollagen in
nativer Form anfällt. Bevorzugt wird ein bovines Kollagen
eingesetzt, das aus Haut oder Sehnen gewonnen wurde und
herkunftsbedingt überwiegend aus Typ-I-Kollagen besteht.

Ein Verfahren zur Herstellung eines geeigneten Ausgangsmaterials ist beispielsweise in der DE-PS 27 30 623 beschrieben.

Wenn für den Wirkstoff-Träger eine erhöhte Formstabilität und eine verlangsamte Bioresorption erwünscht ist,
werden die Polypeptidketten der Kollagen-Matrix zweckmäßigerweise mit einem der üblichen Gerbemittel zusätzlich quervernetzt. Für diesen Zweck geeignete Gerbemittel
sind z. B. Formaldehyd, Glutardialdehyd, Glyoxal, Hexamethylendiisocyanat. Diese werden entweder in wässeriger
Suspension, ggf. unter Beigabe einer Puffersubstanz, z. B.
auf Phosphat-Basis, oder in wässerig-alkoholischer Aufschlämmung mit dem Kollagen umgesetzt. Letzteres Verfahren
wird bevorzugt beim Einsatz von Glyoxal als Vernetzungsmittel angewandt. Geeignete Verfahrensbedingungen für die
Vernetzung mit Glutardialdehyd und Hexamethylendiisocyanat
werden beispielsweise in der DE-PS 27 30 623 bzw. DE-PS
29 43 520 beschrieben.

Zur Beladung mit dem Wirkstoff wird das Trägermaterial
zweckmäßig in Form einer 1 - 2 %igen wässerigen Aufschlämmung vorgelegt, mit der gewünschten Menge an Wirkstoff
beschickt und danach gefriergetrocknet. Durch die Konzentration des Wirkstoffs in der Kollagen-Aufschlämmung kann der Wirkstoffgehalt der Matrix in weiten Grenzen variiert werden. In der Regel wählt man Gehalte von 1 - 10 % Wirkstoff bezogen auf Kollagen-Trockengewicht.

Als Wirkstoffe können im Prinzip alle Stoffe verwendet werden, mit denen Vorgänge im menschlichen oder tierischen Körper beeinflußt werden können. Im Rahmen der vorliegenden Erfindung werden bevorzugt Aminoglycosid-Antibiotika eingelagert, insbesondere wird Gentamycin verwendet. Aminoglycosid-Antibiotika werden in der Regel in Form ihrer leicht wasserlöslichen Salze, wie z. B. dem Sulfat gehandhabt. Eine Kollagen-Matrix, die z. B. mit Gentamycinsulfat beladen ist, gibt den Wirkstoff beim Einbringen in den Körper relativ schnell ab. Es werden dadurch zwar sehr hohe Konzentrationen an Antibiotikum erzeugt, das Depot ist jedoch nach kurzer Zeit erschöpft. Für die Behandlung z. B. einer infizierten Weichteilwunde ist jedoch die Aufrechterhaltung einer wirksamen Konzentration über einen längeren Zeitraum erwünscht.

Es sind schwerlösliche Derivate von Aminoglycosid-Antibiotika bekannt, wie z. B. das Hesperidin-Phosphat, das Lauryl-Sulfat, das Pamoat oder die aus der DOS 32 06 725 bekannten Flavanoid-Phosphate. Eine Beladung der Kollagen-Matrix mit diesen schwerlöslichen Derivaten führt zu einer deutlichen Retardierung der Freisetzung, so daß solche Depots durchaus vorteilhaft eingesetzt werden können.

Es wurde nun jedoch gefunden, daß eine sehr wirksame Retardierung mit einem günstigen Freisetzungsprofil erreicht werden kann, wenn die mit einem leichtlöslichen Salz des Antibiotikums beladene Matrix zusätzlich mit einem sauren Polysaccharid beladen wird. Saure Polysaccharide im Sinne dieser Anmeldung sind polyfunktionelle, physiologisch-verträgliche und im Körper resorbierbare Polysaccharid-Säuren mit Molekulargewichten bis zu mehreren Hunderttausend, die eine Vielzahl von sauren, d. h. ionisierbaren Carboxyl- und Sulfatgruppen enthalten, wie z. B. Pektinsäure, Alginsäure und Carraghenin. Diese Säuren können als Polyanionen fungieren und sind in der Lage die basischen Aminoglycoside zu präzipitieren, so daß diese in eine Art Depot-Form gebracht werden, aus der sie in Abhängigkeit vom pH- und dem Ionen-Milieu der Lösung mehr oder weniger retardiert freigegeben werden. Polysaccharid-Säuren bewirken überdies schon aufgrund ihrer filmbildenden Eigenschaften eine gewisse Retardierung im Sinne einer Mikroverkapselung der Wirkstoffpartikel, so daß sich diese Substanzklasse als besonders vorteilhaft bei der Herstellung von Retardformen für Aminoglycoside erweist, da sie in optimaler Weise verschiedene Wirkprinzipien in sich vereint.

Als polyanionische Verbindungen, die diese Merkmale in sich vereinen, kommen saure Polysaccharide aus der Gruppe pflanzlicher Pektinstoffe, die mit unterschiedlichem Veresterungsgrad beispielsweise im Obst vorkommen, saure Kohlehydrate aus Algen, Flechten und anderen niederen Pflanzen sowie andere, freie Carboxyl- und/oder Monoester der Schwefelsäure enthaltende Polysaccharide aus dem Tier- und Pflanzenreich, wie Chondroitinsulfat, Dermatansulfat, Heparansulfat, Hyaluronsäure u. a. in Frage.

Es kommen aber auch Polysäuren auf Basis oxydierter Zellulose, oxydierter Stärke und andere auf synthetischem Wege mit sauren Funktionen ausgestattete makromolekulare Kohlenhydrate in Frage. Auch natürliche Polyphosphate, wie sie beispielsweise in den Nukleinsäuren vorliegen, sind prinzipiell geeignet.

Alle diese Stoffe sollen im Sinne der vorliegenden Erfindung unter dem Begriff "saure Polysaccharide" zu verstehen sein. Es liegt auf der Hand, daß der Eignungsgrad unterschiedlicher unter diese Definitionen fallender Stoffe auch unterschiedlich ist. Der Fachmann kann jedoch leicht unter den zugänglichen Materialien solche wählen, die bezüglich Biokompatibilität, Resorbierbarkeit, gelierender und filmbildender Wirkung und der Retardwirkung auf medizinische Wirkstoffe geeignet sind. Als besonders gut geeignet haben sich Pektinsäure, Alginsäure und Carraghenin erwiesen.

Die erfindungsgemäßen Polysaccharidsäuren sind in Form ihrer Alkalisalze wasserlöslich und bilden je nach ihrer Konzentration mehr oder minder viskose Lösungen. Bringt man die wässerige Lösung eines solchen makromolekularen Stoffes mit der wässerigen Lösung eines Wirkstoffs mit basischen Gruppen zusammen, so kommt es je nach dem pH-Wert der Lösung zur Präzipitation. Während bei pH 9 in der Regel noch keine Präzipitation zu beachten ist, beginnt diese meist bei etwa pH 8 und verstärkt sich bei niedrigeren pH-Werten.

Aminoglycosid und Polysäure können in gelöster Form bei einem pH-Wert oberhalb von 9,0 in dem als wässerige oder wässerig-alkoholische Suspension vorgelegten Kollagen vereinigt und dann durch pH-Erniedrigung präzipitiert werden. Es kann aber auch eine vorherige Präzipitation vorgenommen werden und das schwerlösliche Konjugat aus dem Aminoglycosid und der betreffenden Polysäure in fester Form in die Kollagen-Suspension eingetragen werden. Schließlich kann vor der Beschickung mit dem Wirkstoff auch noch eine zusätzliche Quervernetzung des Kollagens vorgenommen werden, die zweckmäßig so durchgeführt wird, daß zuerst das Ver-

- 7 -

netzungsmittel und dann, nachdem dieses weitestgehend abreagiert hat, der Wirkstoff zugesetzt wird. Man kann aber auch so verfahren, daß man zunächst ein vliesartiges oder schwammig-poröses Kollagen, das genügend formstabil ist, erzeugt und dieses dann in wässerigem Milieu mit dem Wirkstoff beläd.

In einer bevorzugten Ausführungsform erfolgt die Beschickung mit dem bzw. den Wirkstoffen in einer wässerigen Suspension von Kollagen-Fasern, die dann bei -20° - -40° C eingefroren und anschließend gefriergetrocknet wird. Man erhält je nach Faserlängenverteilung und Vernetzungsgrad des Kollagens ein Depot von vliesartiger oder schwammig-poröser Beschaffenheit. Dieses kann ggf. noch portioniert, sterilisiert und verpackt werden.

Vorzugsweise wird die Präzipitation des Aminoglycosids in der Kollagensuspension selbst vorgenommen, indem man den pH-Wert von zunächst 8,5 - 9,5, bei dem Aminoglycosid und saures Polysaccharid in löslicher Form nebeneinander vorliegen, auf Werte unterhalb pH 7, bevorzugt 4 - 6, erniedrigt.

Die Präzipitation kann aber auch separat erfolgen, so daß in diesem Fall ein bereits vorgefertigtes schwerlösliches Addukt aus Aminoglycosid und saurem Polysaccharid in die Kollagen-Suspension eingetragen wird. Auch kann eine pH-Verschiebung erst nach gefolgter Gefriertrocknung der mit den beiden Komponenten beladenen Kollagenmasse durch anschließendes Begasen mit den Dämpfen einer genügend flüchtigen, physiologisch unbedenkliche Anionen bildenden Säure, wie z. B. Ameisensäure, Essigsäure und andere $C_1$-$C_6$-Carbonsäuren erfolgen.

Schließlich kann man die Präzipitation auch durch Immersion des getrockneten Kollagens in ein organisches, mit Wasser mischbares Lösungsmittel, wie z. B. Aceton oder einen niederen Alkohol wie Methanol, Äthanol oder Propanol, vornehmen, wobei man dem Lösungsmittel die zur Einstellung des gewünschten pH-Wertes erforderliche Menge einer physiologisch akzeptablen Säure zusetzt. Als solche werden Säuren verstanden, die biokompatible Anionen bilden, wie z. B. HCl, $H_2SO_4$, $H_3PO_4$, Ameisensäure, Essigsäure, Milchsäure, Apfelsäure und andere.

Die Menge des sauren Polysaccharids, die zur Präzipitation des Antibiotikums verwendet wird, richtet sich zum einen nach der Menge des Antibiotikums, nach der Anzahl der basischen Gruppen im Antibiotikum und nach der Anzahl der sauren Gruppen im Polysaccharid. Um eine vollständige Umsetzung des Antibiotikums mit dem Polysaccharid zu erreichen, muß die Menge des Polysaccharids so bemessen sein, daß die Anzahl der sauren Gruppen auf jeden Fall höher ist als die Anzahl der basischen Gruppen des Antibiotikums. Nur beispielhaft genannt werden typische sich daraus ergebende Gewichtsverhältnisse von z. B. Gentamycinbase zu Pektinsäure von etwa 1 zu 1,5 bis etwa 1 zu 15 bzw. von Gentamycinbase zu Alginsäure von etwa 1 zu 1,5 bis etwa 1 zu 7,5.

Diese Mengen können gegebenenfalls jedoch auch unter- oder überschritten werden. Falls die Menge des Polysaccharids nicht ausreicht, um die gesamte Menge an Antibiotikum zu binden, wird beim Einbringen des Depots in den Körper zunächst eine relativ hohe Freisetzung erfolgen. Die kann durchaus erwünscht sein, um eine hohe Anfangskonzentration des Antibiotikums zu erzielen, die dann abfällt zu einer wirksamen Dauerdosis.

Andererseits kann durch einen Überschuß an Polysäure eine weitere Retardierung erzielt werden, da das im Fasergeflecht des Kollagens aufquellende Polysaccharid offenbar wie eine Art Ionenaustauscher wirkt, dessen saure Gruppen das Antibiotikum reversibel binden und damit die Wirkstoff-Freisetzung aus dem Träger verlangsamen. Es liegt also in der Hand des Fachmanns, das Freisetzungsprofil in weiten Grenzen zu variieren. Durch einen einfachen Vorversuch kann in jedem Fall festgestellt werden, welche Menge des speziellen Antibiotikums ausreichend ist.

Es wurde bereits erwähnt, daß die vollständige Präzipitation des Wirkstoff-Polysaccharid-Konjugates nicht nur von der Menge des Polysaccharids, sondern insbesondere auch vom pH-Wert abhängig ist. Da bei physiologischem pH-Wert die Löslichkeit des Konjugates noch recht hoch sein kann, kann es von Vorteil sein, der Kollagen-Wirkstoff-Kombination noch eine Puffersubstanz beizugeben, die im schwach sauren Bereich eine hohe Pufferkapazität besitzt. Auf diese Weise kommt auch im physiologischen Milieu der von den Polysacchariden ausgehende retardierende Effekt voll zum Tragen.

Geeignete Puffersysteme sind z. B. Phosphatpuffer nach Sörensen, Na-acetat/Essigsäure, Na-Lactat/Milchsäure, Na-Malat/Äpfelsäure, Na-Salze saurer Aminosäuren, wie Arginin und Lysin, Na-Salze schwacher Peptidsäuren und andere.

Zusätzlich zu den Aminoglycosid-Antibiotika können in die Kollagen-Matrix auch weitere Wirkstoffe eingelagert werden, die eine Antibiotikum-Therapie in sinnvoller Weise ergänzen oder unterstützen können. Auch wenn solche Wirkstoffe keine basischen Gruppen aufweisen, die zu einer Wechselwirkung mit dem sauren Polysaccharid fähig sind, wird auch bei diesen Wirkstoffen allein aufgrund der filmbildenden Eigenschaften und des Geliereffekts der beigegebenen Polysaccharide die freie Diffusion behindert und somit eine verzögerte Freisetzung aus der Kollagen-Matrix beobachtet.

Das erfindungsgemäße Depot kann zu allen lokalen Antibiotika-Behandlungen in den Körper eingebracht werden und kann aufgrund seiner guten Verträglichkeit und der problemlosen Resorption dort verbleiben. Bevorzugt wird es zur lokalen Bekämpfung infizierter Weichteilwunden verwendet. Dabei kommt neben der antibakteriellen Wirkung des Antibiotikums der positive Einfluß des natürlichen Kollagens auf den Heilungsprozeß von Weichgewebsdefekten zum Tragen.

Die erfindungsgemäßen Depots können als Formstücke unterschiedlicher Geometrie gewonnen werden. In der Regel werden flächige, rechteckige Formen mit einer Schichtdicke von etwa 1 mm bis etwa 8 mm und Kantenlängen von etwa 2 cm bis ca. 20 cm bevorzugt.

Beispiel 1:

20 g eines nach dem in der DE-PS 27 30 623 beschriebenen Verfahren gewonnenen Kollagens werden zunächst bei Raumtemperatur in 1 l 0,5 molarer Essigsäure suspendiert. Nach einer Äquilibrierungszeit von ca. 12 h wird der pH-Wert ggf. durch Zugabe von Salzsäure oder Natronlauge korrigiert, so daß ein Wert zwischen 2,5 und 3,5 erreicht wird. Die essigsaure Suspension wird nun homogenisiert, indem mittels eines geeigneten Rührwerkes (z. B. Ultraturrax) unter gleichzeitiger Kühlung die Länge der Kollagenfasern auf Werte unter 10 mm
eingestellt wird.

Nach dem Abpressen der so erhaltenen Kollagenmasse wird
diese in deionisiertem Wasser aufgeschlämmt und erneut
abgepreßt. Dieser Vorgang wird noch zweimal wiederholt.
Das von überschüssiger Essigsäure befreite Kollagen wird
dann unter leichtem Rühren in eine auf 35 °C temperierte
wässerige Lösung von pH 9,5, die 1,2 g Gentamycinsulfat
und 1,2 g Natriumpektat in ca. 900 ml enthält, eingetragen. Nach einer Äquilibrierungszeit von ca. 30 min. versetzt man unter ständiger pH-Kontrolle portionsweise mit
soviel 1-normaler Schwefelsäure bis die breiige Mischung
einen pH zwischen 5 und 6 erreicht hat. Gegebenenfalls
wird das Gesamtvolumen des Ansatzes durch Zugabe von
destilliertem Wasser zu einem Liter ergänzt. Die so erhaltene Masse wird in vorbereitete Gießformen gegossen, bei
-20 - -40 °C schockgefroren und anschließend gefriergetrocknet. Man erhält ein flächiges Wirkstoff-Depot vliesartiger Beschaffenheit mit einem Gehalt von 4 Gew. % Gentamycinbase, welches entsprechend der vorgegebenen Schichthöhe des Kollagen-Breis eine Dicke von 2 mm bis zu etwa
10 mm besitzt. Das Flächengewicht beträgt dann etwa 40 g
pro $m^2$, bzw. 200 g pro $m^2$. Die Wasseraufnahmekapazität
des Wirkstoff-Depots liegt bei etwa 2000 % bezogen auf
Trockenmasse.

Beispiel 2

Eine essigsaure wässerige Aufschlämmung von nativen Kollagen-Fasern wird wie in Beispiel 1 zunächst homogenisiert,
Essigsäure-frei gewaschen und abgepreßt. Diese Masse wird
dann in so viel Isopropylalkohol aufgenommen, daß eine
2 %ige Aufschlämmung in 90 Volumen %igem Isopropanol

vorliegt. Diese wird dann unter Rühren mit insgesamt 0,5 ml 30 %iger wässeriger Glyoxal-Lösung pro 1 l Ansatz bei Raumtemperatur versetzt. Indem die Masse in leichter Bewegung gehalten wird, läßt man das Kollagen 48 Stunden mit dem Gerbemittel reagieren. Dann wird die Masse kurz abgepreßt und wieder in 90 Volumen %igem Isopropanol aufgenommen. Nach dem Abpressen nimmt man die Masse in deionisiertem Wasser auf und preßt diese erneut ab. Die noch feuchte Masse wird dann wie in Beispiel 1 beschrieben in eine vorbereitete Lösung, die Gentamycinsulfat und Natriumpektat im Verhältnis 1:1 enthält, eingetragen. Nach erfolgter pH-Verschiebung auf einen Wert zwischen 5 und 6 wird die Masse bei -20 - -40 °C schockgefroren und lyophilisiert. Man erhält ein vliesartiges Wirkstoff-Depot, das bei Einhaltung der in Beispiel 1 angegebenen Konzentrationen 4 Gew. % Gentamycinbase enthält. Es zeichnet sich gegenüber dem aus Beispiel 1 erhaltenen Produkt durch höhere Formstabilität und verzögerte Bioresorption aus.

Beispiel 3

Die nach Beispiel 1 oder 2 erhaltene Kollagen-Masse wird vor der Beschickung mit Gentamycin in deionisiertem Wasser aufgeschwemmt. Nach Einstellung auf pH 6 mittels Natronlauge bzw. Schwefelsäure wird in die so erhaltene Kollagen-Suspension die berechnete Menge Gentamycin in Form eines vorgefertigten Präzipitats aus Gentamycinsulfat und Natriumpektat eingetragen. Eine 1:1 Fällung der beiden Komponenten enthält etwa 40 Gew. % Gentamycinbase. Nach inniger Vermischung von Wirkstoff-Präzipitat und Kollagen wird die breiige Masse bei -20 - -40 °C schockgefroren und lyophilisiert.

## Beispiel 4

Gemäß Beispiel 1 wird zunächst eine Kollagen-Masse erzeugt, die vor der Beladung mit Wirkstoff einer Quervernetzung mittels Hexamethylendiisocyanat unterzogen wird. Zu diesem Zweck stellt man eine wässerige Aufschlämmung von 2 % bezogen auf Kollagen-Trockenmasse her, korrigiert den pH, wenn nötig, so daß ein Wert zwischen 4 und 5 erreicht wird und kühlt auf 0 °C ab. Dann rührt man einen physiologisch verträglichen nicht-ionischen Emulgator, z. B. Tween 80 ein, dessen Endkonzentration max. 1 % bezogen auf Kollagen-Trockenmasse beträgt. Unter Rühren versetzt man bei 0 °C tropfenweise mit so viel Hexamethylendiisocyanat, daß die Gesamtmenge an Gerbemittel 2 % bezogen auf Trockenmasse Kollagen ausmacht. Man läßt diese Mischung noch 24 Stunden bei 0 °C stehen und preßt dann die erhaltene Masse ab. Die Beschickung mit Gentamycin und die weitere Verarbeitung erfolgt dann nach einem der in den Beispielen 1 - 3 beschriebenen Verfahren. Man erhält ein schwammig-poröses Wirkstoff-Depot, das sich durch ein hohes Maß von Formstabilität auszeichnet.

## Beispiel 5

Nach einem der Beispiele 1 - 4 wird zur Präzipitation des Gentamycins anstelle des Natriumsalzes der Pektinsäure das entsprechende Na-Salz der Alginsäure eingesetzt. Man erhält Wirkstoff-Depots, die denen mit Pektinsäure hergestellten weitgehend entsprechen, sich aber bezüglich der Abgabekinetik des Wirkstoffs von den vorgenannten etwas abheben. Unter sonst gleichen Elutionsbedingungen retardieren die Alginsäure-Präzipitate das Gentamycin etwas stärker als ihre Pektinsäure-Analoga.

Beispiel 6

Nach einem der in den Beispielen 1 - 5 ausgeführten Verfahren wird jeweils die halbe Gentamycinmenge eingesetzt, d. h. 1 l einer 2 %igen Kollagen-Suspension enthalten 0,6 g Gentamycinsulfat bzw. 0,4 g Gentamycinbase in Form eines Alginsäure- oder Pektinsäure-Präzipitats. Die weitere Verarbeitung erfolgt dann analog der für die entsprechenden 4 % Depots angegebenen Verfahrensweise.

Man erhält Gentamycin-Depots mit einem Gehalt von 2 % Gentamycinbase bezogen auf das Trockengewicht der wirkstoffbeladenen Kollagen-Matrix.

Beispiel 7

Nach einem der in den vorgenannten Ausführungsbeispielen dargelegten Verfahren wird das betreffende saure Polysaccharid im Gewichtsverhältnis 2:1 bezogen auf Gentamycinsulfat eingesetzt. Die auf diese Weise hergestellten Wirkstoff-Depots zeichnen sich gegenüber den in den vorausgegangenen Beispielen beschrieben dadurch aus, daß sie eine noch stärkere Retardwirkung auf die Freisetzung des Aminoglykosids ausüben.

Patentansprüche

1. Resorbierbares Wirkstoff-Depot auf Basis von Kollagen, dadurch gekennzeichnet, daß ein rekonstituiertes Kollagen mit Vlies- oder Schwammstruktur mit einem Aminoglykosid-Antibiotikum und einem bioresorbierbaren Polymeren mit polyanionischem Charakter beladen ist.

2. Wirkstoff-Depot nach Anspruch 1, dadurch gekennzeichnet, daß das Kollagen aus Fasern mit einer Länge von max. 10 mm besteht, in denen die native Molekülstruktur weitgehend erhalten ist.

3. Wirkstoff-Depot nach Anspruch 1 oder 2, dadurch gekennzeichnet, daß die Polypeptidketten des Kollagens zusätzlich quervernetzt sind.

4. Wirkstoff-Depot nach Anspruch 3, dadurch gekennzeichnet, daß das Kollagen mit Formaldehyd, Glutardialdehyd, Glyoxal oder Hexamethylendiisocyanat vernetzt ist.

5. Wirkstoff-Depot nach einem der Ansprüche 1 bis 4, dadurch gekennzeichnet, daß als bioresorbierbares Polymer ein saures Polysaccharid enthalten ist.

6. Wirkstoff-Depot nach Anspruch 5, dadurch gekennzeichnet, daß als saures Polysaccharid Pektinsäure oder Alginsäure enthalten ist.

7. Wirkstoff-Depot nach einem der Anprüche 1 bis 6, dadurch gekennzeichnet, daß als Antibiotikum Gentamycin enthalten ist.

8. Wirkstoff-Depot nach einem der Ansprüche 1 bis 7, dadurch gekennzeichnet, daß das Antibiotikum in einer Menge von 1 bis 10 Gewichtsprozent enthalten sind.

9. Wirkstoff-Depot nach einem der Ansprüche 1 bis 8, dadurch gekennzeichnet, daß das bioresorbierbare Polymer gegenüber dem Aminoglykosid-Antibiotikum im Überschuß vorliegt.

10. Verfahren zur Herstellung eines resorbierbaren Antibiotikum-Depots, dadurch gekennzeichnet, daß zu einer Suspension eines rekonstituierten Kollagens, in dem die native Molekülstruktur weitgehend erhalten ist, mit einer Faserlänge von max. 10 mm, ein Aminoglykosid-Antibiotikum und ein bioresorbierbares Polymeres mit polyanionischem Charakter gegeben wird, vor oder nach dieser Zugabe der pH-Wert der Suspension auf einen Wert unterhalb 7 eingestellt wird und die Suspension, ggf. nach Eingießen in Formen, gefriergetrocknet wird.

11. Verfahren nach Anspruch 10, dadurch gekennzeichnet, daß als bioresorbierbares Polymeres ein saures Polysaccharid verwendet wird.

12. Verfahren nach Anspruch 10 oder 11, dadurch gekennzeichnet, daß die Zugabe des Aminoglykosid-Antibiotikums und des sauren Polysaccharids als wasserlösliches Salz zu einer auf pH 8 - 10 eingestellten

0170979

- 3 -

wässerigen Kollagensuspension erfolgt und anschließend eine pH-Verschiebung in einen Bereich von pH 4 bis 7 vorgenommen wird.

13. Verfahren nach einem der Ansprüche 10 bis 12, dadurch gekennzeichnet, daß ein durch Zusammenbringen mit einem sauren Polysaccharid erzeugtes schwerlösliches Konjugat eines Aminoglykosid-Antibiotikums in eine wässerige Kollagen-Suspension von pH 4 bis 7 eingebracht wird.

14. Verfahren nach einem der Ansprüche 11 bis 13, dadurch gekennzeichnet, daß als Salz eines Aminoglykosid-Antibiotikums Gentamycinsulfat und als Salz eines sauren Polysaccharids Natriumalginat oder Natriumpektat verwendet wird.

15. Verfahren nach einem der Ansprüche 10 bis 14, dadurch gekennzeichnet, daß der Kollagen-Suspension vor der Beschickung mit einem Aminoglykosid-Antiobiotikum ein Mittel zur Quervernetzung zugesetzt wird.

16. Verfahren nach Anspruch 15, dadurch gekennzeichnet, daß der Vernetzungsprozeß so geführt wird, daß eine Immobilisierung des Antibiotikums im Sinne einer chemischen Fixierung weitestgehend vermieden wird.

17. Verfahren nach einem der Ansprüche 15 oder 16, dadurch gekennzeichnet, daß die Vernetzung mit Glyoxal in Gegenwart eines niederen Alkohols, vorzugsweise Isopropanol, mit einem Anteil von 80 bis 100 Volumenprozent, vorzugsweise 90 Volumenprozent, vorgenommen wird.

18. Verfahren nach einem der Ansprüche 15 oder 16, dadurch gekennzeichnet, daß als Vernetzungsmittel Hexamethylendiisocyanat in einer Menge von 0 bis 5 %, vorzugsweise 2 %, bezogen auf das Trockengewicht des Kollagens, in Gegenwart eines nichtionogenen Emulgators verwendet wird.

19. Verfahren zur Herstellung eines resorbierbaren Antibiotikum-Depots, dadurch gekennzeichnet, daß man aus rekonstituierten Kollagenfasern, in denen die native Molekülstruktur weitgehend erhalten ist, zunächst vliesartige oder schwammig-poröse Formkörper herstellt, diese mit einer Lösung eines Aminoglykosid-Antibiotikums und eines bioresorbierbaren Polymers mit polyanionischem Charakter tränkt, den pH der Tauchlösung anschließend auf einen Wert unterhalb 7 einstellt und die wirkstoffbeladenen Formkörper gefriertrocknet.

20. Verwendung eines aus nativen Fasern aufgebauten rekonstituierten Kollagens zur Herstellung eines resorbierbaren Wirkstoff-Depots in Form eines Vlieses oder Schwammes.